# EUROPEAN PATENT APPLICATION

(11) **EP 4 679 137 A1**
(43) Date of publication of application: **14.01.2026**
(21) Application number: 23931929.6
(22) Date of filing: 03.04.2023
(51) Int. Cl.: G01S 13/89, A61B 5/00, A61B 5/11, G01S 13/50

(54) **SENSING SYSTEM, RECEPTION DEVICE, CONTROL CIRCUIT, STORAGE MEDIUM, SENSING METHOD, AND RECEIVING METHOD**

(71) Applicant: Mitsubishi Electric Corporation, Chiyoda-ku Tokyo 100-8310 (JP)
(72) Inventor: UMEDA, Shusaku, Tokyo 100-8310 (JP); TAIRA, Akinori, Tokyo 100-8310 (JP); ISHIOKA, Kazuaki, Tokyo 100-8310 (JP)
(74) Representative: Pfenning, Meinig & Partner mbB
(86) International application number: PCT/JP2023/013838
(87) International publication number: WO 2024/209515

(57) **Abstract**

A sensing system (50) includes: a transmitter (10) that includes a plurality of transmitting antenna elements (18), controls timings of generation of a radar signal, generation of a code, and generation of a carrier signal for dividing a frequency band available into a plurality of subbands and periodically switching the subbands, multiplies the radar signal and the code for each of the plurality of the transmitting antenna elements (18), generates a high frequency signal having a bandwidth of the subband using the radar signal multiplied by the code and the carrier signal, and transmits the high frequency signal from each of the plurality of the transmitting antenna elements (18); and a receiver (20) that includes a plurality of receiving antenna elements (22), receives the high frequency signals transmitted from the transmitter (10) and reflected or scattered by a measurement target, generates channel information using the carrier signal, the radar signal, and the code, specifies a position of the measurement target, generates an image of the measurement target by performing focus correction, determines an extraction position for vibration information of the measurement target by using the image, and extracts the vibration information from the channel information.

## Description

### Field

The present disclosure relates to a sensing system, a receiver, a control circuit, a storage medium, a sensing method, and a receiving method, the sensing system measuring a measurement target using electromagnetic waves.

### Background

In recent years, toward the realization of a safe and secure society, there has been an increasing need for a sensing technology that remotely perceives the surrounding environment. Such a sensing technology is used, for example, in an in-vehicle radar or the like for an automobile to perceive a situation of surrounding persons, surrounding vehicles, or the like, and is expected to be used in the future in a digital twin, a cyber-physical system, or the like for collecting environmental information or the like. Meanwhile, as the same sensing application, a heart sound examination using a stethoscope, an electrocardiogram, or the like, an aging investigation of bridges, and the like also have a need to analyze the environment of things from information associated with sound. However, in these methods, due to an impedance mismatch between a measurement target and air, the measurement needs to be performed in direct contact with the measurement target. Therefore, in order to remotely obtain these pieces of information, implementation with a radar system utilizing electromagnetic waves has been considered.

For example, Patent Literature 1 discloses a technique using a multiple input multiple output (MIMO) radar system. The MIMO radar system described in Patent Literature 1 uses a plurality of transmitting elements and a plurality of receiving elements to collect vibration information at a plurality of points on a human body and extracts, from the vibration information collected, vibration on a surface of the human body caused by a heartbeat. The MIMO radar system described in Patent Literature 1 emits a radar signal in a direction of the human body and receives, among reflected signals from the human body, a signal incident from a direction of the directivity that can be achieved by the number of antennas installed on the MIMO radar system, thereby obtaining rough fluctuations in the frequency of the heartbeat.

### Citation List

### Patent Literature

Patent Literature 1: Japanese Patent No. 7001244

### Summary

### Technical Problem

However, in the MIMO radar system described in Patent Literature 1, receiving antennas can be installed in a small number of elements, have wide directivity, and are also oriented in a limited direction, so that it is difficult to always select an optimum beam shape for extracting the vibration information. Therefore, the MIMO radar system described in Patent Literature 1 can only direct a beam in an appropriate direction, perform measurement at a plurality of points, and then perform averaging or selecting, thereby having a problem that the vibration information cannot be extracted at an optimum position.

The present disclosure has been made in view of the above, and an object thereof is to provide a sensing system capable of extracting vibration information of a measurement target with high accuracy.

### Solution to Problem

In order to solve the above-described problems and achieve the object, a sensing system according to the present disclosure includes: a transmitter that includes a plurality of transmitting antenna elements to: control timings of generation of a radar signal, generation of a code for a receiver to separate high frequency signals transmitted from the plurality of the transmitting antenna elements into the high frequency signals transmitted from individual ones of the transmitting antenna elements, and generation of a carrier signal such that a frequency band available is divided into a plurality of subbands and the subbands used for the high frequency signals transmitted from the plurality of the transmitting antenna elements are periodically switched so that an entire range of the frequency band is used; multiply the radar signal and the code for each of the plurality of the transmitting antenna elements; generate the high frequency signal having a bandwidth of the subband using the radar signal multiplied by the code and the carrier signal; and transmit the high frequency signal from each of the plurality of the transmitting antenna elements; and the receiver that includes a plurality of receiving antenna elements to: receive the high frequency signals transmitted from the transmitter and reflected or scattered by a measurement target; generate channel information indicating a state of a channel between the transmitter and the receiver using the carrier signal, the radar signal, and the code; specify a position of the measurement target using the channel information; generate an image of the measurement target by performing focus correction on the measurement target; determine an extraction position for vibration information of the measurement target by using the image; and extract the vibration information of the measurement target from the channel information by using the extraction position.

### Advantageous Effects of Invention

The sensing system according to the present disclosure has an effect of being able to extract the vibration information of the measurement target with high accuracy.

### Brief Description of Drawings

FIG. 1 is a diagram illustrating a general idea of measurement intended to be performed by a sensing system according to a first embodiment.
FIG. 2 is a diagram illustrating an exemplary configuration of the sensing system according to the first embodiment.
FIG. 3 is a graph illustrating an example of high frequency signals transmitted from a transmitter according to the first embodiment.
FIG. 4 is a flowchart illustrating an operation of the sensing system according to the first embodiment.
FIG. 5 is a flowchart illustrating an operation of the transmitter according to the first embodiment.
FIG. 6 is a flowchart illustrating an operation of a receiver according to the first embodiment.
FIG. 7 is a diagram illustrating an exemplary configuration of processing circuitry in a case where the processing circuitry implementing the receiver according to the first embodiment is implemented by a processor and a memory.
FIG. 8 is a diagram illustrating an example of processing circuitry in a case where the processing circuitry implementing the receiver according to the first embodiment includes dedicated hardware.
FIG. 9 is a diagram illustrating an exemplary configuration of a sensing system according to a second embodiment.
FIG. 10 is a flowchart illustrating an operation of the sensing system according to the second embodiment.
FIG. 11 is a flowchart illustrating an operation of a receiver according to the second embodiment.
FIG. 12 is a diagram illustrating a general idea of measurement intended to be performed by a sensing system according to a third embodiment.
FIG. 13 is a first diagram illustrating an exemplary configuration of the sensing system according to the third embodiment.
FIG. 14 is a second diagram illustrating an exemplary configuration of the sensing system according to the third embodiment.
FIG. 15 is a diagram illustrating a general idea of measurement intended to be performed by a sensing system according to a fourth embodiment.
FIG. 16 is a diagram illustrating a general idea of measurement intended to be performed by a sensing system according to a fifth embodiment.

### Description of Embodiments

Hereinafter, a sensing system, a receiver, a control circuit, a storage medium, a sensing method, and a receiving method according to embodiments of the present disclosure will be described in detail with reference to the drawings.

### First Embodiment.

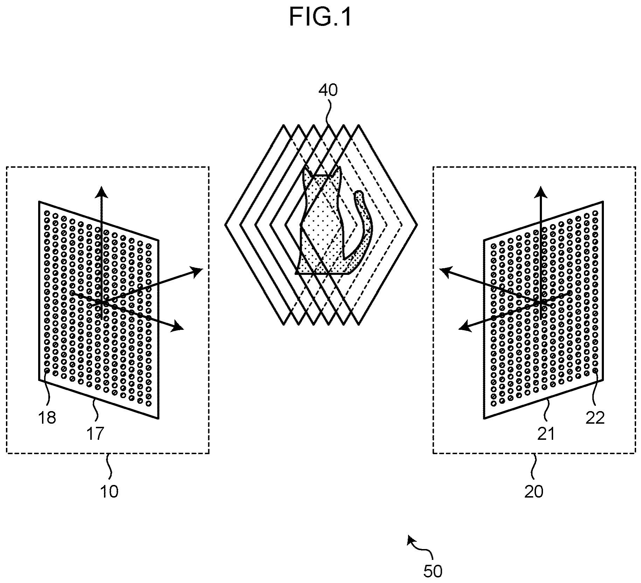

FIG. 1 is a diagram illustrating a general idea of measurement intended to be performed by a sensing system 50 according to a first embodiment. The sensing system 50 is a system that includes a transmitter 10 and a receiver 20 and measures a measurement target 40. In the sensing system 50, the transmitter 10 emits radio waves from a transmitting array 17 including a plurality of transmitting antenna elements 18 to the measurement target 40, and the receiver 20 receives reflected waves, scattered waves, or the like from the measurement target 40 by a receiving array 21 including a plurality of receiving antenna elements 22, whereby the measurement target 40 is measured. In the first embodiment, the transmitter 10 performs transmission of high frequency signals as the emission of the radio waves. In the transmitter 10, the transmitting array 17 includes N_{T} pieces of the transmitting antenna elements 18 as the plurality of the transmitting antenna elements 18. In the receiver 20, the receiving array 21 includes N_{R} pieces of the receiving antenna elements 22 as the plurality of the receiving antenna elements 22. As will be described later, the sensing system 50 extracts information of the measurement target 40 from N_{T}×N_{R} or more pieces of channel information formed between the N_{T} pieces of the transmitting antenna elements 18 and the N_{R} pieces of the receiving antenna elements 22.

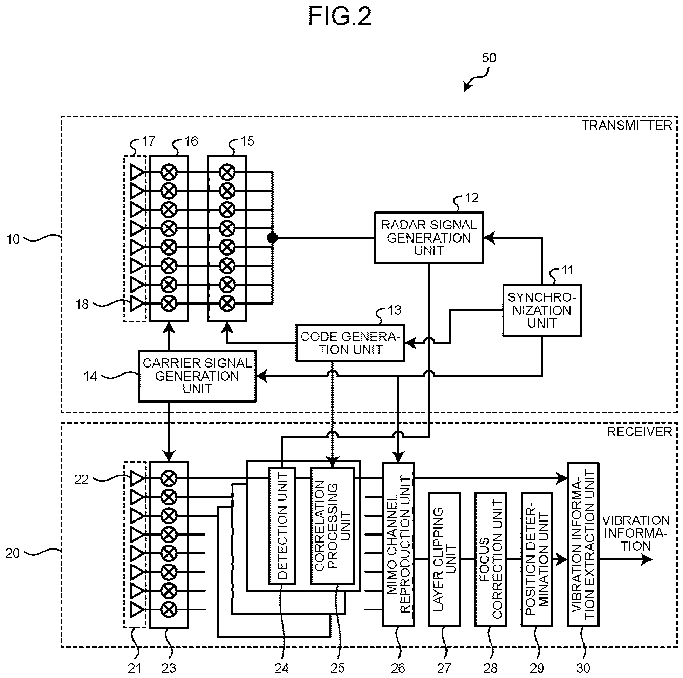

FIG. 2 is a diagram illustrating an exemplary configuration of the sensing system 50 according to the first embodiment. As described above, the sensing system 50 includes the transmitter 10 and the receiver 20. The transmitter 10 includes a synchronization unit 11, a radar signal generation unit 12, a code generation unit 13, a carrier signal generation unit 14, an encoding unit 15, a high frequency signal generation unit 16, and the transmitting array 17. As described above, the transmitting array 17 includes the N_{T} pieces of the transmitting antenna elements 18.

The synchronization unit 11 adjusts the timing of operation of each unit in the transmitter 10 and the receiver 20. The synchronization unit 11 controls the timings of generation of a radar signal by the radar signal generation unit 12, generation of a code by the code generation unit 13, and generation of a carrier signal by the carrier signal generation unit 14 in the transmitter 10. The radar signal generation unit 12 generates the radar signal at baseband or an intermediate frequency. The radar signal is a periodic broadband signal as described later. The code generation unit 13 generates the codes for the receiver 20 to separate the high frequency signals transmitted from the transmitting array 17, which includes the plurality of the transmitting antenna elements 18, into the high frequency signals transmitted from the individual transmitting antenna elements 18. The carrier signal generation unit 14 generates a reference carrier for generating a final high frequency signal. As will be described later, the carrier signal generation unit 14 generates the carrier signals such that a frequency band available for use by the transmitter 10 is divided into a plurality of subbands and the subbands used for the high frequency signals transmitted from the plurality of the transmitting antenna elements 18 are periodically switched so that an entire range of the frequency band is used.

The encoding unit 15 performs, for each of the plurality of the transmitting antenna elements 18, code multiplication in which the radar signal generated by the radar signal generation unit 12 is multiplied by the code generated by the code generation unit 13. For each of the transmitting antenna elements 18, the high frequency signal generation unit 16 generates the high frequency signal to be transmitted from the transmitting antenna element 18 using the signal obtained by multiplying the radar signal by the code in the encoding unit 15 and the reference carrier generated in the carrier signal generation unit 14. The high frequency signal generation unit 16 is, for example, an up-converter or a multiplier and generates the high frequency signal having a bandwidth of the subband by using the radar signal multiplied by the code and the carrier signal, thereby allowing the high frequency signal to be transmitted from each of the plurality of the transmitting antenna elements 18. In the transmitting array 17, the transmitting antenna elements 18 transmit the high frequency signals generated by the high frequency signal generation unit 16.

The receiver 20 includes the receiving array 21, a signal conversion unit 23, a detection unit 24, a correlation processing unit 25, a MIMO channel reproduction unit 26, a layer clipping unit 27, a focus correction unit 28, a position determination unit 29, and a vibration information extraction unit 30. As described above, the receiving array 21 includes the N_{R} pieces of the receiving antenna elements 22.

In the receiving array 21, the receiving antenna elements 22 receive the high frequency signals transmitted from the transmitter 10, the high frequency signals being the reflected waves reflected by the measurement target 40 or the scattered waves scattered by the measurement target 40. That is, the receiving antenna elements 22 receive the reflected waves or the scattered waves of the high frequency signals transmitted from the transmitter 10. Note that the receiving antenna elements 22 can also directly receive the high frequency signals transmitted from the transmitter 10 depending on the positional relationship, orientation relationship, or the like between the transmitting antenna elements 18 of the transmitter 10 and the receiving antenna elements 22 of the receiver 20. For each of the receiving antenna elements 22, the signal conversion unit 23 converts the high frequency signals received by the receiving antenna element 22 into baseband or intermediate frequency signals, that is, down-converts the high frequency signals. The signal conversion unit 23 is, for example, a down-converter and converts the high frequency signals received by the plurality of the receiving antenna elements 22 into received signals in the frequency band of the radar signals, which are used for the generation of the high frequency signals by the transmitter 10, by using the carrier signals used for the generation of the high frequency signals by the transmitter 10.

The detection unit 24 is disposed for each of the receiving antenna elements 22 and detects the baseband or intermediate frequency received signals, which are obtained after conversion by the signal conversion unit 23, using the radar signals generated by the radar signal generation unit 12 of the transmitter 10, thereby obtaining received information. The received information is the reflected waves or the scattered waves of the high frequency signals received by the receiving antenna elements 22 and includes the high frequency signals transmitted from the plurality of the transmitting antenna elements 18. Note that the receiver 20 may obtain the received information by mixing, with a mixer, the baseband or intermediate frequency received signals obtained after conversion by the signal conversion unit 23. Hereinafter, a case where the detection unit 24 performs the detection will be described. The correlation processing unit 25 is disposed for each of the receiving antenna elements 22 and performs correlation processing on the received information, which is detected and obtained by the detection unit 24, using the codes generated by the code generation unit 13 of the transmitter 10, thereby separating the received information into the signals from the transmitting antenna elements 18 of the transmitter 10, that is, separating the received signals received by the plurality of the receiving antenna elements 22 into the signals of the individual transmitting antenna elements 18 transmitted from the transmitter 10 for each of the receiving antenna elements 22.

The MIMO channel reproduction unit 26 reproduces a state of the channels between the transmitter 10 and the receiver 20 by using the signals separated for the individual transmitting antenna elements 18 for each of the receiving antenna elements 22 by the correlation processing unit 25, and generates MIMO channel information indicating the state of the channels. The MIMO channel reproduction unit 26 generates the MIMO channel information for each frequency bin to be described later. In the following description, the MIMO channel reproduction unit may be simply referred to as a channel reproduction unit, and the MIMO channel information may be simply referred to as channel information. The layer clipping unit 27 specifies the position of the measurement target 40 using the MIMO channel information. The layer clipping unit 27 clips the measurement target 40 by a specific curved surface from the MIMO channel information reproduced by the MIMO channel reproduction unit 26. Specifically, the layer clipping unit 27 specifies the position of the measurement target 40 by extracting, from the MIMO channel information, reflection point information of a layer corresponding to a distance in a depth direction of the measurement target 40 as viewed from the plurality of the receiving antenna elements 22. The focus correction unit 28 performs focus correction on the measurement target 40 whose position has been specified, and generates and outputs an image that is image information of the measurement target 40. As the focus correction on the measurement target 40, the focus correction unit 28 performs focus correction corresponding to the position of the layer on the reflection point information extracted.

The position determination unit 29 uses the image of the measurement target 40 generated by the focus correction unit 28, and determines an extraction position for vibration information of the measurement target 40. The extraction position for the vibration information is, for example, an optimum position for extracting the vibration information to be measured, that is, a desired piece of the vibration information, and is a position indicated by a layer and a focus where the vibration information is easily extracted. For example, in a case where the measurement target 40 is a human body with the vibration information to be measured, that is, a desired piece of the vibration information being a heartbeat of the human body as the measurement target 40, the extraction position for the vibration information is a position where the heartbeat can be measured in the human body. A relationship between the measurement target 40 and the extraction position for the vibration information may be set in advance by a user or a manufacturer of the sensing system 50, or may be set by the user of the sensing system 50 each time the measurement is performed. The vibration information extraction unit 30 extracts, from the MIMO channel information generated by the MIMO channel reproduction unit 26, the vibration information of the measurement target 40 by using the extraction position determined by the position determination unit 29. For example, the vibration information extraction unit 30 weights each piece of the channel information on the basis of the MIMO channel information, generates a virtual beam shape for acquiring reflection information from the direction of the position indicated by the extraction position, and extracts the vibration information from information obtained, that is, the reflection information from the direction of the extraction position. The vibration information extraction unit 30 can extract the vibration information from, for example, a cycle of change in the reflection information obtained. As described above, in the sensing system 50, the transmitter 10 does not perform actual beam control, but the receiver 20 performs virtual beam control.

The operation of the sensing system 50 will be described. In the transmitter 10, the synchronization unit 11 controls, on the basis of a frame configuration described later, a timing of radar signal generation by the radar signal generation unit 12, a timing of code generation by the code generation unit 13, and a timing of carrier frequency switching by the carrier signal generation unit 14.

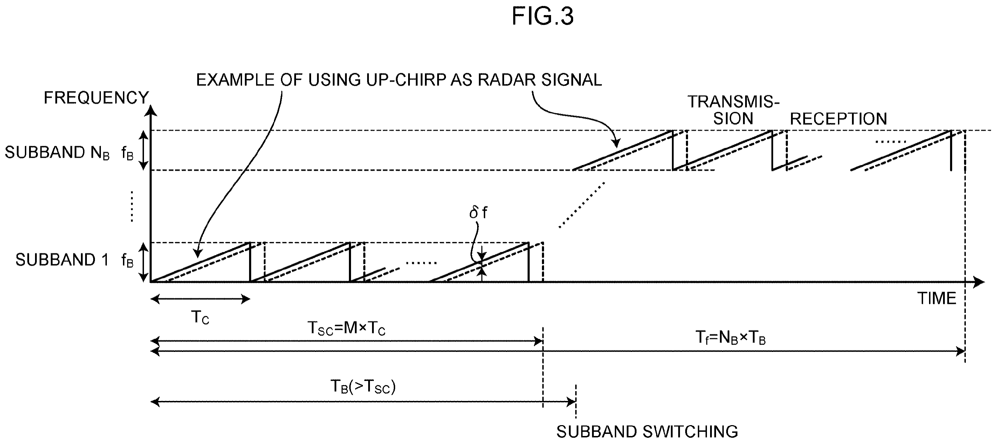

FIG. 3 is a graph illustrating an example of the high frequency signals transmitted from the transmitter 10 according to the first embodiment. FIG. 3 illustrates the example in which an up-chirp is used as the radar signal generated by the radar signal generation unit 12, but the radar signal is not limited to the up-chirp and need only be a signal with the spectrum spreading over an entire specific frequency band, such as an up-down chirp, a Zadoff-Chu (ZC) sequence, a pseudo noise (hereinafter referred to as PN) signal, an orthogonal frequency division multiplexing (OFDM) signal, a frequency step signal that is a signal whose frequency changes stepwise in a time direction, a general spread signal, or the like. The frequency band available to the sensing system 50 is divided into partial N_{B} bands each having a bandwidth f_{B}. In FIG. 3, the N_{B} bands are denoted as subband 1 to subband N_{B}. These bands, that is, the subband 1 to the subband N_{B} may partially overlap each other.

The radar signal generation unit 12 generates, as the radar signal, a periodic broadband signal represented by a chirp signal in each subband. At this time, the synchronization unit 11 instructs the radar signal generation unit 12 on a timing of the beginning of each period. The period of the broadband signal is set to be, when "A" is an integer, 1/A of a chip period T_{C}. That is, the code generation unit 13 generates the code such that the chip period T_{C} of the code is an integer multiple of the period of the radar signal. FIG. 3 illustrates an example when A=1. The code generation unit 13 generates the code having a code length of M chips to be used by each of the transmitting antenna elements 18. As illustrated in FIG. 3, the code period is T_{SC}=M×T_{C}. At this time, the synchronization unit 11 instructs the code generation unit 13 on a timing of the beginning of the code period. The encoding unit 15 performs code multiplication in which the radar signal generated by the radar signal generation unit 12 is multiplied by the code generated by the code generation unit 13. The code is generally expressed as ±1. The encoding unit 15 performs the multiplication processing as phase modulation, amplitude modulation, frequency modulation, or a combination thereof.

The transmitter 10 transmits the radar signal for at least the code period T_{SC} in each subband, but may transmit the radar signal for a duration longer than or equal to the code period T_{SC}, such as for a time period of "T_{B}" illustrated in FIG. 3, by repeatedly using the code. The time period of "T_{B}" illustrated in FIG. 3 is a subband switching period.

After completing the transmission of the radar signal for the subband switching period T_{B} in one subband, the transmitter 10 switches the frequency, that is, switches the subband. The carrier signal generation unit 14 generates the carrier signal for converting the radar signal encoded by the encoding unit 15 into the high frequency signal in each subband. The carrier signal generation unit 14 receives an instruction from the synchronization unit 11 and generates the carrier signal that is appropriate for switching the subband every subband switching period T_{B}. The high frequency signal generation unit 16 uses the carrier signal generated by the carrier signal generation unit 14 to convert the radar signal encoded by the encoding unit 15 into the high frequency signal in each subband, and transmits the high frequency signal from the transmitting antenna element 18 of the transmitting array 17. A period in which the transmitter 10 transmits the radar signals from the subband 1 to the subband N_{B}, that is, over the entire frequency band of the N_{B} subbands is defined as a frame period T_{f}=N_{B}×T_{B}. Assuming that the high frequency signals from the subband 1 to the subband N_{B} illustrated in FIG. 3 correspond to one frame, the transmitter 10 completes one measurement by transmission of one frame, that is, in one frame period T_{f}.

Note that the codes generated by the code generation unit 13 are used to identify the signals between the transmitting antenna elements 18. Therefore, the code generation unit 13 generates what is called orthogonal codes or quasi-orthogonal codes having a small cross-correlation between the transmitting antenna elements 18. Note that, as the code generated by the code generation unit 13 and used for the identification between the transmitting antenna elements 18, an M sequence, a Gold code, a Walsh-Hadamard code, a PN sequence, and the like are known, but the code is not limited thereto as long as the code has high orthogonality.

In the sensing system 50, the transmitter 10 and the receiver 20 are synchronized in time and frequency, and operate according to the same instruction from the synchronization unit 11. Note that the sensing system 50 may have a bistatic configuration in which the transmitter 10 and the receiver 20 are disposed at physically separated positions. In this case, the transmitter 10 and the receiver 20 can be synchronized using not only a wired connection but also a global positioning system (GPS), various wireless links, or the like. For example, the sensing system 50 may be in a mode in which the carrier signal generation unit 14 is disposed independently of the transmitter 10 and the receiver 20, and a base signal and a reference signal for matching frequencies are shared by the transmitter 10 and the receiver 20. Here, as illustrated in FIG. 2, the configuration in which the functional units of the transmitter 10 and the receiver 20 are connected by wire is used as an example to describe operations of the transmitter 10 and the receiver 20.

In the receiver 20, the high frequency signals emitted from the transmitter 10 to the measurement target 40 and reflected or scattered by the measurement target 40 are received by the receiving antenna elements 22 of the receiving array 21. The signal conversion unit 23 uses the carrier signal corresponding to each subband generated by the carrier signal generation unit 14 to convert the high frequency signals received by the receiving antenna elements 22 of the receiving array 21 into the baseband or intermediate frequency signals, that is, down-convert the high frequency signals. The detection unit 24 is disposed for each of the receiving antenna elements 22 and detects the baseband or intermediate frequency signals, which are obtained after conversion by the signal conversion unit 23, using the radar signals generated by the radar signal generation unit 12 of the transmitter 10, thereby obtaining the received information. The received information includes the signals transmitted from all the N_{T} pieces of the transmitting antenna elements 18 as received by a specific one of the receiving antenna elements 22.

The correlation processing unit 25 uses the codes generated by the code generation unit 13 of the transmitter 10 to perform correlation processing on the received information detected and obtained by the detection unit 24, thereby separating the received information into the signals from the transmitting antenna elements 18 of the transmitter 10. In the receiver 20, the processing of the correlation processing unit 25 is performed for each of the receiving antenna elements 22, so that N_{T}×N_{R} pieces of the channel information of each subband are obtained. Note that the detection processing in the detection unit 24 varies depending on what kind of signal is used as the radar signal, and thus detailed description thereof is omitted here. In the first embodiment, the detection processing in the detection unit 24 may be a versatile processing method.

How wide the bandwidth f_{B} of the subband is set depends on the frequency band used by the sensing system 50, various architectures thereof, and the like. In particular, in a case where the sensing system 50 uses an ultrahigh frequency band such as a terahertz band, there is a high possibility that a large fluctuation in frequency characteristics occurs in the band of the subband. Therefore, the sensing system 50 may divide the bandwidth f_{B} of the subband into a plurality of frequency bins and calculate the channel information. Generally, the frequency bin is set to a bandwidth in which a frequency fluctuation in the band is assumed to be constant. In the receiver 20, the MIMO channel reproduction unit 26 divides each subband into N_{F} frequency bins, and calculates the channel information formed between the transmitting array 17 of the transmitter 10 and the receiving array 21 of the receiver 20 with the measurement target 40 sandwiched therebetween as illustrated in FIG. 1. As a result, the MIMO channel reproduction unit 26 can integrate the information of all the subbands to obtain the MIMO channel information including N_{T}×N_{R}×N_{F}×N_{B} elements. In the following description, the frequency bin may be referred to as a frequency bin.

As described above, the transmitter 10 transmits the high frequency signals by dividing the frequency band available into the plurality of the subbands and periodically switching the subbands used for the high frequency signals transmitted from the plurality of the transmitting antenna elements 18 so that the entire range of the frequency band is used. At this time, as the MIMO channel information, the MIMO channel reproduction unit 26 generates the MIMO channel information, the number of pieces of which is equal to the multiplication product of the number of the plurality of the transmitting antenna elements 18 included in the transmitter 10, the number of the plurality of the receiving antenna elements 22 included in the receiver 20, the number of the subbands, and the number of the frequency bins obtained when the bandwidth of the subband is divided into the plurality of the frequency bins.

Note that in order to spatially generate N_{T}×N_{R} pieces of the MIMO channel information, here, the technique of using N_{T}×N_{R} pieces of the real antenna elements has been described. On the other hand, using an approximation technique, that is, interpolation, extrapolation, or the like can also generate N_{T}×N_{R} pieces of the MIMO channel information while reducing the number of the real antenna elements. Such processing itself is a general technique, and thus detailed description thereof is omitted here.

As with the detection processing, different techniques are used for the generation of the MIMO channel information depending on the type of the radar signal, the detection method, and the like, and thus the technique of generating the MIMO channel information is not limited here. For example, in a case where the chirp signal as illustrated in FIG. 3 is used as the radar signal, the receiver 20 obtains the carrier signal having a frequency difference δf corresponding to a distance to the reflection point after the detection. In a case where a plurality of the reflection points is present, the carrier signals having a plurality of frequencies are superimposed. From this information, the receiver 20 calculates a phase, amplitude characteristics, and the like of each frequency bin.

The MIMO channel information obtained includes all the reflection point information of the measurement target 40. The layer clipping unit 27 clips only the reflection point information on a specific curved surface determined from the placement of the transmitting antenna elements 18 and the receiving antenna elements 22. Note that the specific curved surface may be a specific flat surface. The focus correction unit 28 can obtain a tomographic image of a portion of the measurement target 40 by focusing on the curved surface. Note that the processing of the layer clipping unit 27 and the processing of the focus correction unit 28 are performed in no particular order. The position determination unit 29 uses the tomographic image acquired from the focus correction unit 28 to determine an optimum extraction position for extracting the vibration information of the measurement target 40. The optimum extraction position includes set values of the layer and focus, or the like. The vibration information extraction unit 30 weights each piece of the channel information of the MIMO channel information using the extraction position determined by the position determination unit 29, generates the virtual beam shape for acquiring the reflection information from the direction of the position indicated by the extraction position, and extracts the vibration information in units of the time of the code period T_{SC} from the information obtained. The reflection information from the direction of the position indicated by the extraction position may be the reflection information of only the extraction position or the reflection information within a prescribed range including the extraction position, depending on the vibration information to be measured, that is, the vibration information desired. Note that the number of the subbands used in the stage of determining the extraction position for the vibration information may be different from the number of the subbands used in the stage of continuously acquiring the vibration information. That is, the transmitter 10 and the receiver 20 may use different numbers of the subbands in the stage of determining the extraction position for the vibration information and the stage of extracting the vibration information. This is because a case is assumed in which spatial resolution including the depth direction is required in the stage of determining the extraction position, whereas information such as predetermined azimuth angle and elevation angle need only be acquired in the stage of acquiring the vibration information. For example, in a case where reflection occurs only on a skin surface, separation of information in the depth direction is not required. In acquiring the vibration information, there is a need to increase resolution in the time direction, in other words, to capture vibration up to high frequency. Therefore, in the stage of acquiring the vibration information, it is possible to reduce the number of the subbands to be used and speed up the cycle of measurement.

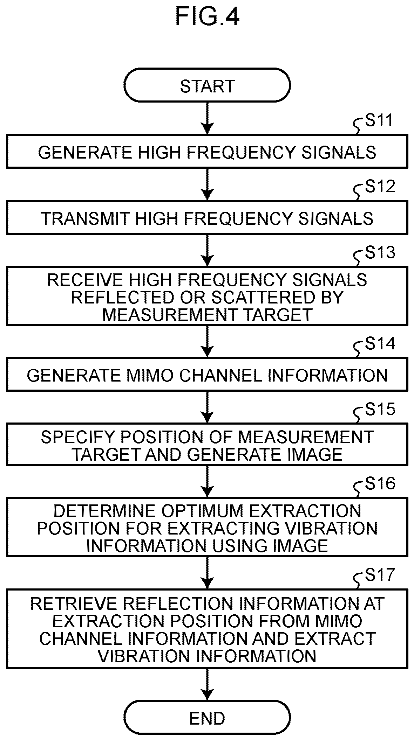

FIG. 4 is a flowchart illustrating the operation of the sensing system 50 according to the first embodiment. In the sensing system 50, the transmitter 10 generates the high frequency signals (step S11) and transmits the high frequency signals from the transmitting array 17 toward the measurement target 40 (step S12). The receiver 20 receives the high frequency signals transmitted from the transmitter 10 and reflected or scattered by the measurement target 40 (step S13), generates the MIMO channel information indicating the state of the channels between the transmitter 10 and the receiver 20 using the carrier signals, the radar signals, and the codes (step S14), and specifies the position of the measurement target 40 using the MIMO channel information to generate the image of the measurement target 40 by performing focus correction on the measurement target 40 (step S15). The receiver 20 determines the optimum extraction position for extracting the vibration information of the measurement target 40 using the image generated (step S16), and retrieves the reflection information at the determined extraction position from the MIMO channel information to extract the vibration information of the measurement target 40 (step S17).

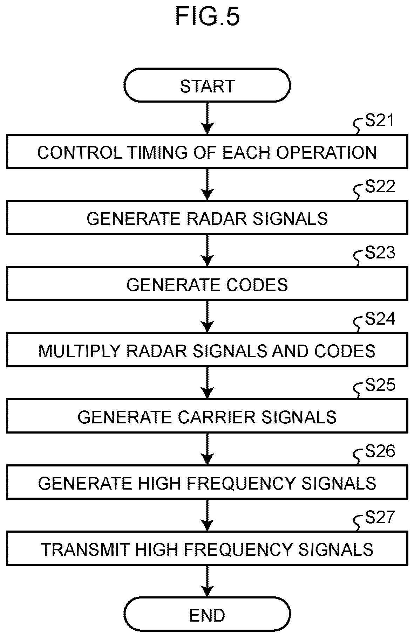

FIG. 5 is a flowchart illustrating the operation of the transmitter 10 according to the first embodiment. The flowchart illustrated in FIG. 5 illustrates details of the operations in step S11 and step S12 of the flowchart illustrated in FIG. 4. In the transmitter 10, the synchronization unit 11 controls the timings of the generation of the radar signal in the radar signal generation unit 12, the generation of the code in the code generation unit 13, and the generation of the carrier signal in the carrier signal generation unit 14, that is, controls the timing of each operation (step S21). The radar signal generation unit 12 under the control of the synchronization unit 11 generates the radar signals as the broadband signals (step S22). The code generation unit 13 under the control of the synchronization unit 11 generates the codes for the receiver 20 to separate the high frequency signals transmitted from the plurality of the transmitting antenna elements 18 into the high frequency signals transmitted from the individual transmitting antenna elements 18 (step S23). The encoding unit 15 multiplies the radar signal by the code for each of the plurality of the transmitting antenna elements 18 (step S24). The carrier signal generation unit 14 under the control of the synchronization unit 11 generates the carrier signals such that the frequency band available for use by the transmitter 10 is divided into the plurality of the subbands and the subbands used for the high frequency signals transmitted from the plurality of the transmitting antenna elements 18 are periodically switched so that the entire range of the frequency band is used (step S25). The high frequency signal generation unit 16 generates the high frequency signal having the bandwidth of the subband by using the radar signal multiplied by the code and the carrier signal (step S26). The plurality of the transmitting antenna elements 18 transmits the high frequency signals (step S27).

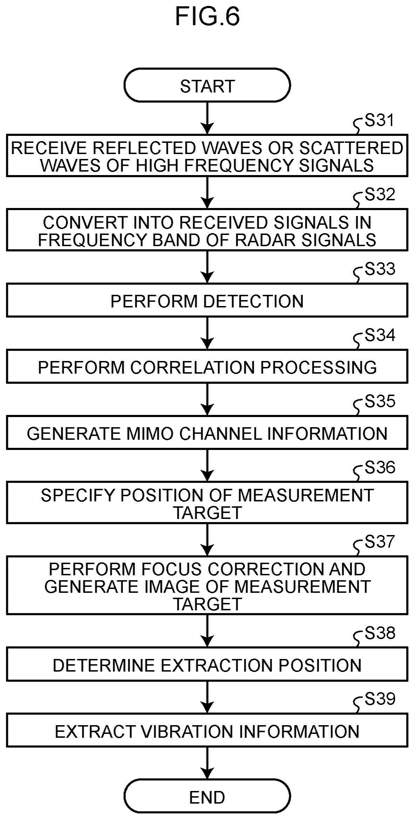

FIG. 6 is a flowchart illustrating the operation of the receiver 20 according to the first embodiment. The flowchart illustrated in FIG. 6 illustrates details of the operations from step S13 to step S17 of the flowchart illustrated in FIG. 4. In the receiver 20, the plurality of the receiving antenna elements 22 receives the reflected waves or the scattered waves of the high frequency signals transmitted from the transmitter 10, which includes the plurality of the transmitting antenna elements 18, and reflected or scattered by the measurement target 40 (step S31). The signal conversion unit 23 converts the reflected waves or the scattered waves of the high frequency signals received by the plurality of the receiving antenna elements 22 into the received signals in the frequency band of the radar signals, which are used for the generation of the high frequency signals by the transmitter 10, by using the carrier signals used for the generation of the high frequency signals by the transmitter 10 (step S32). The detection unit 24 detects the received signals using the radar signals generated by the transmitter 10, and obtains the received information that is the reflected waves or the scattered waves of the high frequency signals received by the receiving antenna elements 22 and includes the high frequency signals transmitted from the plurality of the transmitting antenna elements 18 (step S33). The correlation processing unit 25 performs the correlation processing on the received information by using the codes used when the radar signals are encoded by the transmitter 10, and separates the received signals into the individual signals of the transmitting antenna elements 18 transmitted from the transmitter 10 for each of the receiving antenna elements 22 (step S34). The MIMO channel reproduction unit 26 uses the separated signals to generate the MIMO channel information indicating the state of the channels between the transmitter 10 and the receiver 20 (step S35). The layer clipping unit 27 specifies the position of the measurement target 40 using the MIMO channel information (step S36). The focus correction unit 28 performs focus correction on the measurement target 40 whose position has been specified, and generates the image of the measurement target 40 (step S37). The position determination unit 29 uses the image of the measurement target 40 generated by the focus correction unit 28, and determines the extraction position for the vibration information of the measurement target 40 (step S38). The vibration information extraction unit 30 extracts, from the MIMO channel information generated by the MIMO channel reproduction unit 26, the vibration information of the measurement target 40 by using the extraction position determined by the position determination unit 29 (step S39).

Next, a hardware configuration of each device in the sensing system 50 will be described. In the receiver 20, the receiving array 21 includes the plurality of the receiving antenna elements 22. The signal conversion unit 23, the detection unit 24, the correlation processing unit 25, the MIMO channel reproduction unit 26, the layer clipping unit 27, the focus correction unit 28, the position determination unit 29, and the vibration information extraction unit 30 are implemented by processing circuitry. The processing circuitry may include a memory and a processor that executes a program stored in the memory, or may include dedicated hardware. The processing circuitry is also called a control circuit.

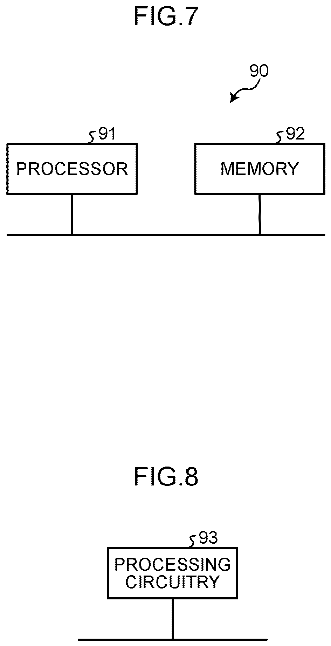

FIG. 7 is a diagram illustrating an exemplary configuration of processing circuitry 90 in a case where the processing circuitry implementing the receiver 20 according to the first embodiment is implemented by a processor 91 and a memory 92. The processing circuitry 90 illustrated in FIG. 7 is the control circuit and includes the processor 91 and the memory 92. In the case where the processing circuitry 90 incudes the processor 91 and the memory 92, the functions of the processing circuitry 90 are implemented by software, firmware, or a combination of software and firmware. The software or firmware is described as the program and stored in the memory 92. The processing circuitry 90 implements the functions by the processor 91 reading and executing the program stored in the memory 92. That is, the processing circuitry 90 includes the memory 92 for storing the program, the execution of which results in the execution of the processing of the receiver 20. It can also be said that this program is a program for causing the receiver 20 to execute the functions implemented by the processing circuitry 90. This program may be provided by a storage medium storing the program, or may be provided by other means such as a communication medium.

It can also be said that the above program is a program for causing the receiver 20 to execute: a signal conversion step in which the signal conversion unit 23 converts the reflected waves or the scattered waves of the high frequency signals received by the plurality of the receiving antenna elements 22 into the received signals in the frequency band of the radar signals, which are used for the generation of the high frequency signals by the transmitter 10, by using the carrier signals used for the generation of the high frequency signals by the transmitter 10; a detection step in which the detection unit 24 detects the received signals using the radar signals and obtains the received information that is the reflected waves or the scattered waves of the high frequency signals received by the receiving antenna elements 22 and includes the high frequency signals transmitted from the plurality of the transmitting antenna elements 18; a correlation processing step in which the correlation processing unit 25 performs correlation processing on the received information by using the codes used when the radar signals are encoded by the transmitter 10, and separates the received signals into the individual signals of the transmitting antenna elements 18 transmitted from the transmitter 10 for each of the receiving antenna elements 22; a channel reproduction step in which the MIMO channel reproduction unit 26 uses the signals separated and generates the MIMO channel information indicating the state of the channels between the transmitter 10 and the receiver 20; a layer clipping step in which the layer clipping unit 27 specifies the position of the measurement target 40 by extracting, from the MIMO channel information, the reflection point information of the layer corresponding to the distance in the depth direction of the measurement target 40 as viewed from the plurality of the receiving antenna elements 22; a focus correction step in which the focus correction unit 28 generates the image of the measurement target 40 by performing focus correction on the measurement target 40 after the position of the measurement target 40 is specified; a position determination step in which the position determination unit 29 determines the extraction position for the vibration information of the measurement target 40 by using the image; and a vibration information extraction step in which the vibration information extraction unit 30 extracts the vibration information of the measurement target 40 from the MIMO channel information by using the extraction position.

Here, the processor 91 is, for example, a central processing unit (CPU), a processing unit, an arithmetic unit, a microprocessor, a microcomputer, a digital signal processor (DSP), or the like. The memory 92 corresponds to, for example, a non-volatile or volatile semiconductor memory such as a random access memory (RAM), a read only memory (ROM), a flash memory, an erasable programmable ROM (EPROM), or an electrically EPROM (EEPROM (registered trademark)), a magnetic disk, a flexible disk, an optical disk, a compact disc, a mini disc, a digital versatile disc (DVD), or the like.

FIG. 8 is a diagram illustrating an example of processing circuitry 93 in a case where the processing circuitry implementing the receiver 20 according to the first embodiment includes the dedicated hardware. The processing circuitry 93 illustrated in FIG. 8 corresponds to, for example, a single circuit, a complex circuit, a programmed processor, a parallel-programmed processor, an application specific integrated circuit (ASIC), a field programmable gate array (FPGA), or a combination thereof. The processing circuitry may be implemented partly by the dedicated hardware and partly by the software or firmware. The processing circuitry can thus implement the functions described above by the dedicated hardware, the software, the firmware, or a combination thereof.

While the hardware configuration of the receiver 20 has been described, the transmitter 10 has a similar hardware configuration. In the transmitter 10, the transmitting array 17 includes the plurality of the transmitting antenna elements 18. The synchronization unit 11, the radar signal generation unit 12, the code generation unit 13, the carrier signal generation unit 14, the encoding unit 15, and the high frequency signal generation unit 16 are implemented by processing circuitry. The processing circuitry may include a memory and a processor that executes a program stored in the memory, or may include dedicated hardware. The processing circuitry is also called a control circuit.

As described above, according to the present embodiment, in the sensing system 50, the receiver 20 generates the image of the measurement target 40, determines the optimum position for extracting the vibration information of the measurement target 40, and extracts the vibration information from the MIMO channel information on the basis of the information of the position determined. As a result, the sensing system 50 can extract the vibration information of the measurement target 40 with high accuracy without performing control on the transmission wave. In the sensing system 50, the transmitter 10 does not perform actual beam control, but the receiver 20 performs virtual beam control. Note that in the present embodiment, in the sensing system 50, the transmitter 10 includes the plurality of the transmitting antenna elements 18, but even in the case of a single antenna, a similar effect can be obtained by similar operation.

### Second Embodiment.

In the first embodiment, in a case where the sensing system 50 attempts to extract vibration information of a region with a small phase fluctuation, vibration information to be interference may be included besides the vibration information to be extracted. For example, in a case where the vibration information to be extracted is heartbeat information, there is vibration information, such as vibration information due to respiration or the like, that is distributed in a region with a small phase fluctuation as with the heartbeat information. A second embodiment will describe a case where, in a sensing system, a receiver performs filtering on vibration information obtained.

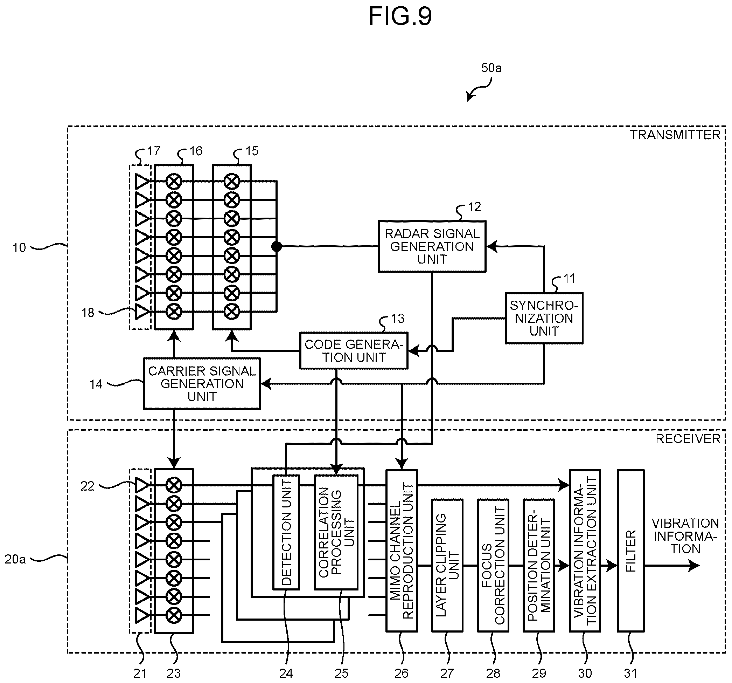

FIG. 9 is a diagram illustrating an exemplary configuration of a sensing system 50a according to the second embodiment. The sensing system 50a includes the transmitter 10 and a receiver 20a. The transmitter 10 is the same as the transmitter 10 of the first embodiment illustrated in FIG. 2. The receiver 20a is obtained by adding a filter 31 to the receiver 20 illustrated in FIG. 2. The filter 31 performs filtering on the vibration information extracted by the vibration information extraction unit 30 to extract, from the vibration information extracted by the vibration information extraction unit 30, the vibration information about vibration having a desired period. Specifically, for the vibration information extracted by the vibration information extraction unit 30, the filter 31 filters the vibration information of a component vibrating with a period corresponding to a frequency lower than a specified frequency. The filter 31 is, for example, a filter such as a high-pass filter or a band-pass filter. The receiver 20a uses the filter 31 to be able to extract, from the vibration information extracted by the vibration information extraction unit 30, only the vibration information at a frequency higher than the specified frequency. Slow fluctuation, that is, vibration at a low frequency includes, for example, respiratory information, body motion information, and the like in terms of vital information. The receiver 20a filters these pieces of information to be able to extract only the vibration information, such as of pulsation, whose phase change is abrupt compared to respiration and body motion and which fluctuates at a higher frequency.

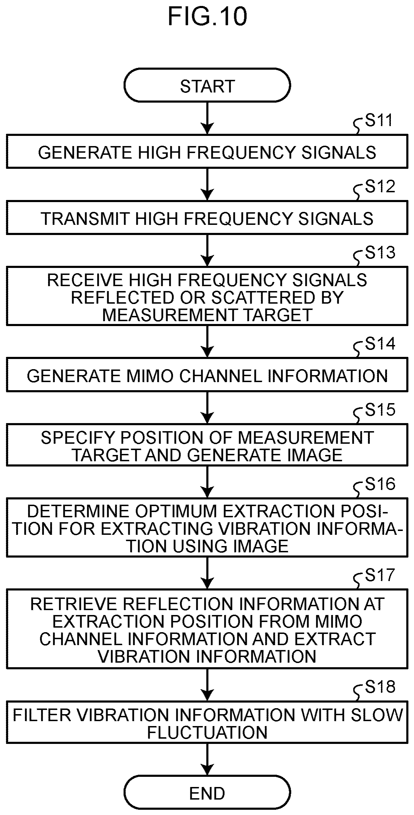

FIG. 10 is a flowchart illustrating an operation of the sensing system 50a according to the second embodiment. In the flowchart illustrated in FIG. 10, the operation from step S11 to step S17 is the same as the operation from step S11 to step S17 of the flowchart for the sensing system 50 of the first embodiment illustrated in FIG. 4. For the vibration information extracted, the receiver 20a filters the vibration information with a fluctuation slower than a specified speed (step S18).

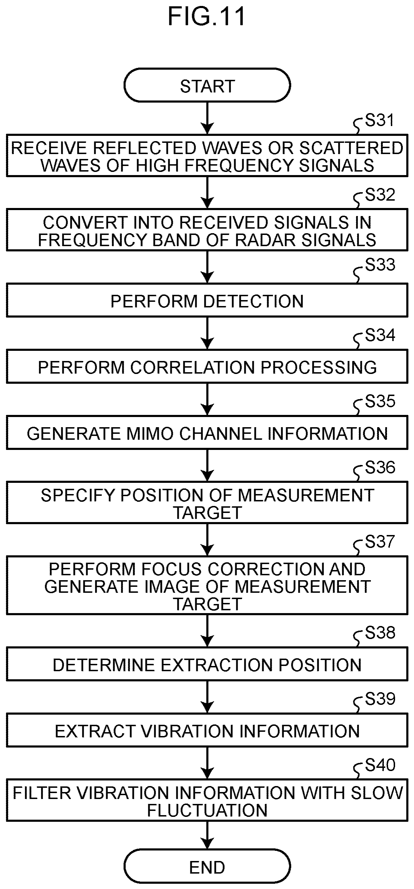

FIG. 11 is a flowchart illustrating an operation of the receiver 20a according to the second embodiment. The flowchart illustrated in FIG. 11 illustrates details of the operation from step S13 to step S18 of the flowchart illustrated in FIG. 10. Also, in the flowchart illustrated in FIG. 11, the operation from step S31 to step S39 is the same as the operation from step S31 to step S39 of the flowchart for the receiver 20 of the first embodiment illustrated in FIG. 6. For the vibration information extracted by the vibration information extraction unit 30, the filter 31 filters the vibration information with the fluctuation slower than the specified speed (step S40).

As described above, according to the present embodiment, in the sensing system 50a, the receiver 20a uses the filter 31 to perform filtering on the vibration information extracted by the vibration information extraction unit 30. As a result, the receiver 20a can extract, from the vibration information extracted by the vibration information extraction unit 30, only the vibration information with a period corresponding to a frequency higher than the specified frequency.

### Third Embodiment.

A third embodiment will describe a case where a sensing system includes a plurality of the transmitters 10 and a plurality of receivers.

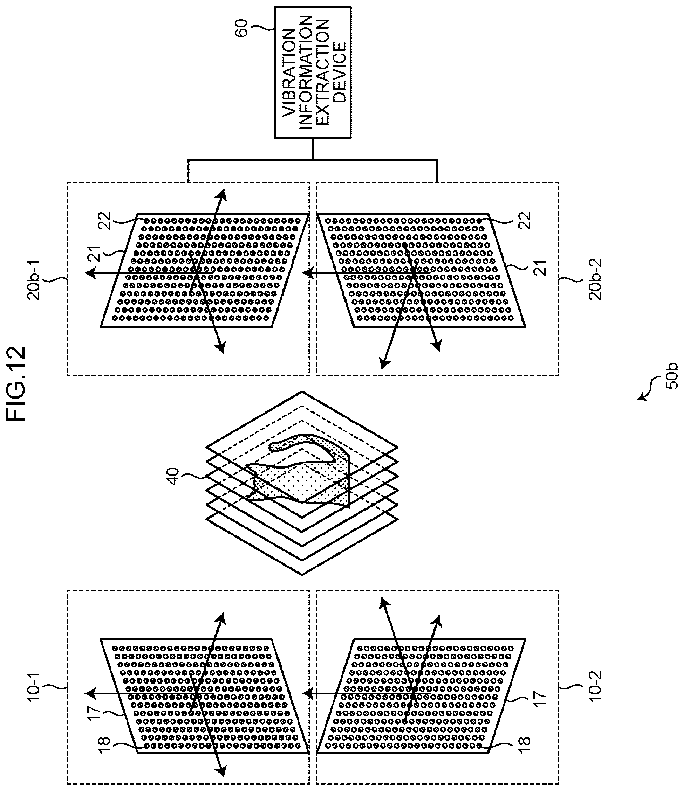

FIG. 12 is a diagram illustrating a general idea of measurement intended to be performed by a sensing system 50b according to the third embodiment. The sensing system 50b is a system that includes a plurality of transmitters 10-1 and 10-2, a plurality of receivers 20b-1 and 20b-2, and a vibration information extraction device 60, and measures the measurement target 40. That is, in the third embodiment, the plurality of the transmitters 10-1 and 10-2, the plurality of the receivers 20b-1 and 20b-2, and the vibration information extraction device 60 constitute the sensing system 50b. The transmitters 10-1 and 10-2 each have a configuration similar to that of the transmitter 10 of the first embodiment, and may be referred to as the transmitter 10 when not distinguished from each other. Similarly, the receivers 20b-1 and 20b-2 may be referred to as a receiver 20b when not distinguished from each other. In FIG. 12, the plurality of the transmitters 10 is represented as the transmitters 10-1 and 10-2, and the plurality of the receivers 20b is represented as the receivers 20b-1 and 20b-2, but the number of the plurality of the transmitters 10 and the number of the plurality of the receivers 20b may be three or more. The sensing system 50b is a system in which a plurality of sets of the transmitters 10 and the receivers 20b is located nearby, and the plurality of the transmitters 10 performs sensing by emitting radio waves at the same time. In the third embodiment, the transmitting arrays 17 of the plurality of the transmitters 10 operate nearby, so that a mechanism for suppressing interference between the transmitting arrays 17 is required.

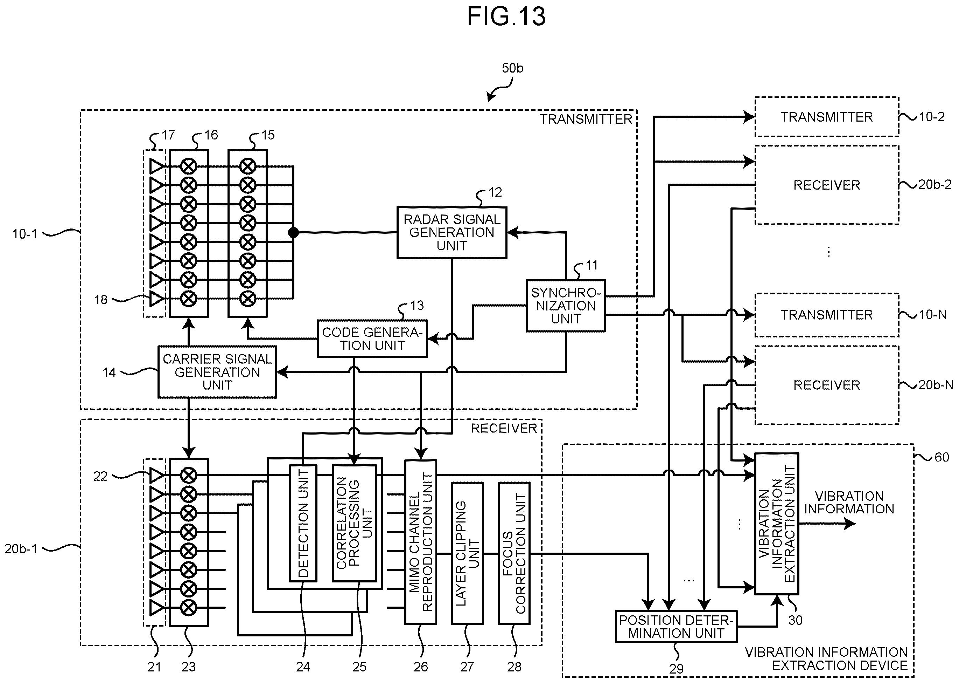

FIG. 13 is a first diagram illustrating an exemplary configuration of the sensing system 50b according to the third embodiment. In the example of FIG. 13, the sensing system 50b includes the transmitter 10-1 to a transmitter 10-N as N pieces of the transmitters 10, the receiver 20b-1 to a receiver 20b-N as N pieces of the receivers 20b, and the vibration information extraction device 60. The configuration of the transmitter 10-1 illustrated in FIG. 13 is similar to the configuration of the transmitter 10 illustrated in FIG. 2. However, the synchronization unit 11 of the transmitter 10-1 also instructs the other transmitters 10-2 to 10-N and the receivers 20b-1 to 20b-N, which operate simultaneously, on the timing of operation of each component. The configuration of the receiver 20b-1 illustrated in FIG. 13 is obtained by removing the position determination unit 29 and the vibration information extraction unit 30 from the configuration of the receiver 20 illustrated in FIG. 2. In the third embodiment, the vibration information extraction device 60 includes the position determination unit 29 and the vibration information extraction unit 30. That is, in the sensing system 50b, the receivers 20b-1 to 20b-N share the position determination unit 29 and the vibration information extraction unit 30.

The transmitters 10-2 to 10-N may each have a configuration similar to the configuration of the transmitter 10-1, or have a configuration obtained by removing the synchronization unit 11 from the configuration of the transmitter 10-1. In the case where the transmitters 10-2 to 10-N each have the configuration similar to the configuration of the transmitter 10-1, for example, the synchronization unit 11 of the transmitter 10-1 serves as a master, and the synchronization unit 11 of each of the transmitters 10-2 to 10-N serves as a slave so that the synchronization unit 11 of the transmitter 10-1 instructs the synchronization unit 11 of each of the transmitters 10-2 to 10-N on the timing of operation of each component. As a result, on the basis of the instruction from the synchronization unit 11 of the transmitter 10-1, the synchronization unit 11 of each of the transmitters 10-2 to 10-N can instruct each component therein on the timing of operation. In the case where the transmitters 10-2 to 10-N each have the configuration obtained by removing the synchronization unit 11 from the configuration of the transmitter 10-1, the synchronization unit 11 of the transmitter 10-1 directly instructs each component in the transmitters 10-2 to 10-N on the timing of operation. The following description will be given using, as an example, the case where the transmitters 10-2 to 10-N each have the configuration obtained by removing the synchronization unit 11 from the configuration of the transmitter 10-1, that is, a case where the transmitters 10-2 to 10-N each do not include the synchronization unit 11. Note that the receivers 20b-2 to 20b-N each have a configuration similar to the configuration of the receiver 20b-1.

The operation of the sensing system 50b will be described. The operation of the transmitter 10-1 is similar to the operation of the transmitter 10 of the first embodiment. A transmitter 10-K receives the instruction from the synchronization unit 11 of the transmitter 10-1, and operates the radar signal generation unit 12 and the code generation unit 13 of the transmitter 10-K at the same timing as the timing at which the transmitter 10-1 operates the radar signal generation unit 12 and the code generation unit 13 of the transmitter 10-1. Note that "K" is an integer satisfying 2≤K≤N. At this time, the code generation unit 13 of the transmitter 10-K generates a code having a small correlation among the transmitters 10-1 to 10-N. Examples of the code generated by the code generation unit 13 of the transmitter 10-K include an M sequence, a Gold code, a Walsh-Hadamard code, a PN sequence, and the like, but the code is not limited thereto as long as the code has high orthogonality. In the sensing system 50b, the carrier signal generation units 14 and the high frequency signal generation units 16 of the transmitters 10-1 to 10-N perform the same operation in all the transmitters 10-1 to 10-N and switch the carrier frequency at the same timing.

As described above, the radar signal generation units 12, the code generation units 13, and the carrier signal generation units 14 in the plurality of the transmitters 10-1 to 10-N operate in synchronization. Moreover, the code generation units 13 in the plurality of the transmitters 10-1 to 10-N generate the codes having a low correlation among the plurality of the transmitters 10-1 to 10-N. The code generation units 13 in the plurality of the transmitters 10-1 to 10-N generate orthogonal codes or quasi-orthogonal codes as the codes having a low correlation among the plurality of the transmitters 10-1 to 10-N. That is, the plurality of the transmitters 10-1 to 10-N operates in synchronization with one another in terms of the generation of the radar signals, the generation of the codes, and the generation of the carrier signals, and generates the codes having a low correlation among the plurality of the transmitters 10-1 to 10-N.

The operation of the receiver 20b-1 is similar to the operation performed by the receiving array 21, the signal conversion unit 23, the detection unit 24, the correlation processing unit 25, the MIMO channel reproduction unit 26, the layer clipping unit 27, and the focus correction unit 28 among the components included in the receiver 20 of the first embodiment. The operation of each of the receivers 20b-2 to 20b-N is similar to that of the receiver 20b-1. The correlation processing unit 25 of a receiver 20b-K performs correlation processing using the code used in the transmitter 10-K to extract only the signal from the transmitter 10-K while suppressing the signals from the other transmitters 10, thereby being able to generate the MIMO channel information. Thus, the correlation processing unit 25 of each of the plurality of the receivers 20b-1 to 20b-N performs correlation processing using the code used in the corresponding one of the transmitters 10.

The vibration information extraction device 60 uses an image generated by each of the receivers 20b-1 to 20b-N to determine the extraction position for the vibration information of the measurement target 40 and extracts, from the MIMO channel information generated by the receivers 20b-1 to 20b-N, the vibration information of the measurement target 40 using the extraction position. Specifically, in the vibration information extraction device 60, the position determination unit 29 performs an operation similar to that of the position determination unit 29 included in the receiver 20 of the first embodiment, but determines the extraction position for the vibration information of the measurement target 40 using the images acquired from the receivers 20b-1 to 20b-N, that is, N pieces of images. Moreover, in the vibration information extraction device 60, the vibration information extraction unit 30 performs an operation similar to that of the vibration information extraction unit 30 included in the receiver 20 of the first embodiment, but extracts the vibration information of the measurement target 40 using the extraction position determined by the position determination unit 29 from the MIMO channel information acquired from the receivers 20b-1 to 20b-N, that is, N pieces of the MIMO channel information.

In the third embodiment, the MIMO channel reproduction unit 26 of each of the receivers 20b-1 to 20b-N outputs the MIMO channel information generated to the vibration information extraction device 60 together with the layer clipping unit 27. Moreover, the focus correction unit 28 of each of the receivers 20b-1 to 20b-N outputs the generated image to the vibration information extraction device 60.

Note that the third embodiment has been described assuming that the number of the transmitters 10 and the number of the receivers 20b are the same, but in the sensing system 50b, the number of the transmitters 10 and the number of the receivers 20b do not necessarily have to be the same. For example, in a case where the transmitters 10-1 and 10-2 as two of the transmitters 10 and a receiver 20bA as one of the receiver 20b are operated, the receiver 20bA can generate the MIMO channel information corresponding to two directions by the correlation processing unit 25 collectively using the codes used in the transmitters 10-1 and 10-2, and can generate and output an image corresponding to the two directions. Also, in a case where a transmitter 10A as one of the transmitter 10 and the receivers 20b-1 and 20b-2 as two of the receivers 20b are operated, the receivers 20b-1 and 20b-2 both use the code used in the transmitter 10A to be able to obtain images corresponding to their respective positional relationships. The position determination unit 29 uses the image generated by one or more of the receivers 20b to determine the receiver 20b and position information suitable for extracting the vibration information of the measurement target 40. On the basis of the receiver 20b and the position information determined by the position determination unit 29, the vibration information extraction unit 30 extracts, from the MIMO channel information of the receiver 20b, the vibration information of the measurement target 40 using the position information.

As described above, according to the present embodiment, in the sensing system 50b, the plurality of the transmitters 10-1 to 10-N simultaneously transmits the high frequency signals, and the plurality of the receivers 20b-1 to 20b-N performs measurement and imaging in parallel. The vibration information extraction device 60 uses the MIMO channel information and the images generated by the plurality of the receivers 20b-1 to 20b-N to determine the optimum position for extracting the vibration information of the measurement target 40, and extracts the vibration information from the MIMO channel information on the basis of the information of the position determined. As a result, the sensing system 50b can achieve imaging from a plurality of directions faster, extract the vibration information from the optimum position, and extract the vibration information of the measurement target 40 with higher accuracy.

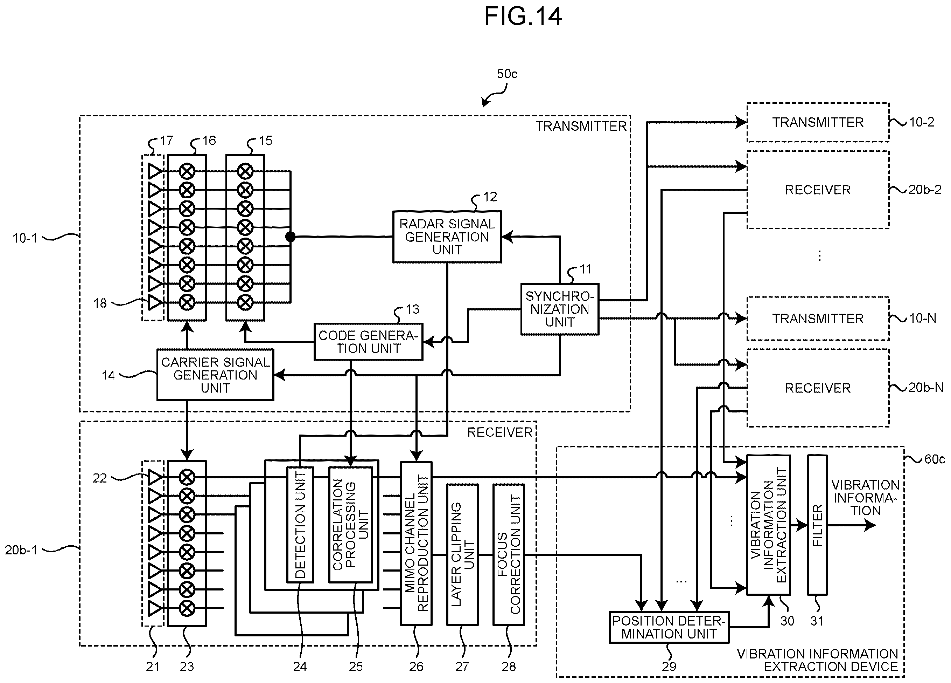

Note that the vibration information extraction device 60 of the sensing system 50b can include the filter 31 described in the second embodiment. FIG. 14 is a second diagram illustrating an exemplary configuration of a sensing system 50c according to the third embodiment. The sensing system 50c includes the transmitters 10-1 to 10-N, the receivers 20b-1 to 20b-N, and a vibration information extraction device 60c. The vibration information extraction device 60c is obtained by adding the filter 31 to the vibration information extraction device 60 illustrated in FIG. 13. The filter 31 is similar to the filter 31 described in the second embodiment. As a result, in the sensing system 50c, the vibration information extraction device 60c can extract only the vibration information at a frequency higher than a specified frequency from the vibration information extracted by the vibration information extraction unit 30.

### Fourth Embodiment.

In the first embodiment, the sensing system 50 includes one piece of the measurement target 40. A fourth embodiment will describe a case where the sensing system 50 includes a plurality of the measurement targets 40.

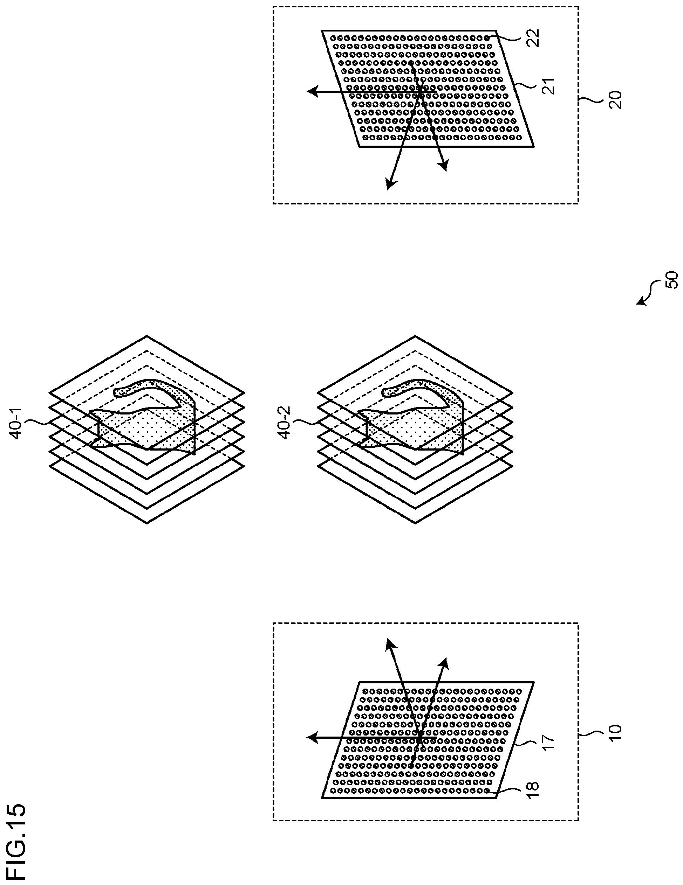

FIG. 15 is a diagram illustrating a general idea of measurement intended to be performed by the sensing system 50 according to the fourth embodiment. In the example of FIG. 15, as the plurality of the measurement targets 40, two of the measurement targets 40 are represented as measurement targets 40-1 and 40-2. In the fourth embodiment, the configurations of the transmitter 10 and the receiver 20 are the same as the configurations of the transmitter 10 and the receiver 20 of the first embodiment illustrated in FIG. 2. In the fourth embodiment, an image generated by the focus correction unit 28 of the receiver 20 includes the measurement targets 40-1 and 40-2, that is, the measurement targets 40.

In the receiver 20, the position determination unit 29 determines, for each of the measurement targets 40, an optimum extraction position for extracting the vibration information of each of the measurement targets 40 from the image of the measurement targets 40-1 and 40-2, that is, the measurement targets 40. In the receiver 20, the vibration information extraction unit 30 extracts, from the MIMO channel information, the vibration information of each of the measurement targets 40 by using the extraction position for each of the measurement targets 40 determined by the position determination unit 29. The position determination unit 29 and the vibration information extraction unit 30 may have higher processing loads than in the first embodiment due to the increase in the number of the measurement targets 40, but the operation itself on each of the measurement targets 40 is similar to the operation in the first embodiment.

As described above, according to the present embodiment, in the sensing system 50, when the plurality of the measurement targets 40 is present, the receiver 20 determines, for each of the measurement targets 40, the optimum extraction position for extracting the vibration information of each of the measurement targets 40 from the image generated and extracts, from the MIMO channel information, the vibration information of each of the measurement targets 40 using the extraction position determined. As a result, even when the plurality of the measurement targets 40 is present, the receiver 20 can extract the vibration information of the plurality of the measurement targets 40 with high accuracy.

Note that although not illustrated, the sensing system 50a in which the receiver 20a includes the filter 31 can also extract the vibration information for a plurality of the measurement targets 40.

### Fifth Embodiment.

In the third embodiment, the sensing system 50b includes one piece of the measurement target 40. A fifth embodiment will describe a case where the sensing system 50b includes a plurality of the measurement targets 40.

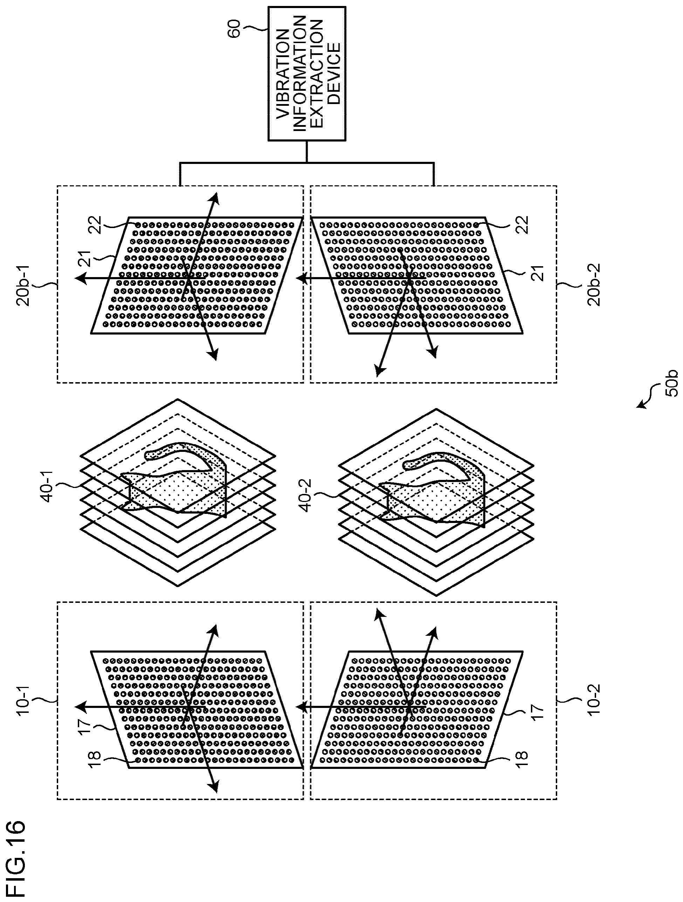

FIG. 16 is a diagram illustrating a general idea of measurement intended to be performed by the sensing system 50b according to the fifth embodiment. In the example of FIG. 16, as the plurality of the measurement targets 40, two of the measurement targets 40 are represented as the measurement targets 40-1 and 40-2. Note that the sensing system 50b includes, as in the third embodiment as illustrated in FIG. 13, N pieces of the transmitters 10, N pieces of the receivers 20b, and the vibration information extraction device 60. In the fifth embodiment, the configurations of the transmitter 10-1, the receiver 20b-1, the vibration information extraction device 60, and the like are the same as the configurations of the transmitter 10-1, the receiver 20b-1, the vibration information extraction device 60, and the like of the third embodiment illustrated in FIG. 13, respectively. In the fifth embodiment, an image generated by the focus correction unit 28 of each of the receivers 20b-1 to 20b-N includes the measurement targets 40-1 and 40-2, that is, the measurement targets 40.

In the vibration information extraction device 60, the position determination unit 29 determines, for each of the measurement targets 40, an optimum extraction position for extracting the vibration information of each of the measurement targets 40 from the image of the measurement targets 40-1 and 40-2, that is, the measurement targets 40. In the vibration information extraction device 60, the vibration information extraction unit 30 extracts, from the MIMO channel information, the vibration information of each of the measurement targets 40 by using the extraction position for each of the measurement targets 40 determined by the position determination unit 29. The position determination unit 29 and the vibration information extraction unit 30 may have higher processing loads than in the third embodiment due to the increase in the number of the measurement targets 40, but the operation itself on each of the measurement targets 40 is similar to the operation in the third embodiment.

As described above, according to the present embodiment, in the sensing system 50b, when the plurality of the measurement targets 40 is present, the vibration information extraction device 60 determines, for each of the measurement targets 40, the optimum extraction position for extracting the vibration information of each of the measurement targets 40 from the image generated by each of the receivers 20b-1 to 20b-N and extracts, from the MIMO channel information, the vibration information of each of the measurement targets 40 using the extraction position determined. As a result, even when the plurality of the measurement targets 40 is present, the vibration information extraction device 60 can extract the vibration information of the plurality of the measurement targets 40 with high accuracy.

Note that although not illustrated, the sensing system 50c in which the vibration information extraction device 60c includes the filter 31 can also extract the vibration information for a plurality of the measurement targets 40.

The configurations illustrated in the above embodiments each merely illustrate an example so that another known technique can be combined, the embodiments can be combined together, or the configurations can be partially omitted and/or modified without departing from the scope of the present disclosure.

### Reference Signs List

10, 10-1 to 10-N transmitter; 11 synchronization unit; 12 radar signal generation unit; 13 code generation unit; 14 carrier signal generation unit; 15 encoding unit; 16 high frequency signal generation unit; 17 transmitting array; 18 transmitting antenna element; 20, 20a, 20b-1 to 20b-N receiver; 21 receiving array; 22 receiving antenna element; 23 signal conversion unit; 24 detection unit; 25 correlation processing unit; 26 MIMO channel reproduction unit; 27 layer clipping unit; 28 focus correction unit; 29 position determination unit; 30 vibration information extraction unit; 31 filter; 40, 40-1, 40-2 measurement target; 50, 50a, 50b, 50c sensing system; 60, 60c vibration information extraction device; 90, 93 processing circuitry; 91 processor; 92 memory.

## Claims

1. A sensing system comprising:
a transmitter that includes a plurality of transmitting antenna elements to: control timings of generation of a radar signal, generation of a code for a receiver to separate high frequency signals transmitted from the plurality of the transmitting antenna elements into the high frequency signals transmitted from individual ones of the transmitting antenna elements, and generation of a carrier signal such that a frequency band available is divided into a plurality of subbands and the subbands used for the high frequency signals transmitted from the plurality of the transmitting antenna elements are periodically switched so that an entire range of the frequency band is used; multiply the radar signal and the code for each of the plurality of the transmitting antenna elements; generate the high frequency signal having a bandwidth of the subband using the radar signal multiplied by the code and the carrier signal; and transmit the high frequency signal from each of the plurality of the transmitting antenna elements; and
the receiver that includes a plurality of receiving antenna elements to: receive the high frequency signals transmitted from the transmitter and reflected or scattered by a measurement target; generate channel information indicating a state of a channel between the transmitter and the receiver using the carrier signal, the radar signal, and the code; specify a position of the measurement target using the channel information; generate an image of the measurement target by performing focus correction on the measurement target; determine an extraction position for vibration information of the measurement target by using the image; and extract the vibration information of the measurement target from the channel information by using the extraction position.

2. The sensing system according to claim 1, wherein
the receiver performs filtering on the vibration information to extract, from the vibration information, vibration information about vibration having a desired period.

3. The sensing system according to claim 1 or 2, wherein
the receiver determines the extraction position optimal for extracting the vibration information of a plurality of the measurement targets from the image for each of the measurement targets, and extracts, from the channel information, the vibration information of the plurality of the measurement targets by using the extraction position for each of the measurement targets.

4. A sensing system comprising a plurality of transmitters and a plurality of receivers, the sensing system comprising:
the plurality of the transmitters that each includes a plurality of transmitting antenna elements to: control timings of generation of a radar signal, generation of a code for the receivers to each separate high frequency signals transmitted from the plurality of the transmitting antenna elements into the high frequency signals transmitted from individual ones of the transmitting antenna elements, and generation of a carrier signal such that a frequency band available is divided into a plurality of subbands and the subbands used for the high frequency signals transmitted from the plurality of the transmitting antenna elements are periodically switched so that an entire range of the frequency band is used; multiply the radar signal and the code for each of the plurality of the transmitting antenna elements; generate the high frequency signal having a bandwidth of the subband using the radar signal multiplied by the code and the carrier signal; and transmit the high frequency signal from each of the plurality of the transmitting antenna elements;
the plurality of the receivers that each includes a plurality of receiving antenna elements to: receive the high frequency signals transmitted from the transmitters and reflected or scattered by a measurement target; generate channel information indicating a state of a channel between the transmitters and the receivers using the carrier signal, the radar signal, and the code; specify a position of the measurement target using the channel information; and generate an image of the measurement target by performing focus correction on the measurement target; and
a vibration information extraction device to determine an extraction position for vibration information of the measurement target by using the image, and extract the vibration information of the measurement target from the channel information by using the extraction position.

5. The sensing system according to claim 4, wherein
the vibration information extraction device performs filtering on the vibration information to extract, from the vibration information, vibration information about vibration having a desired period.

6. The sensing system according to claim 4 or 5, wherein
the vibration information extraction device determines the extraction position optimal for extracting the vibration information of a plurality of the measurement targets from the image for each of the measurement targets, and extracts, from the channel information, the vibration information of the plurality of the measurement targets by using the extraction position for each of the measurement targets.

7. The sensing system according to any one of claims 1 to 6, wherein
the transmitter and the receiver use different numbers of the subbands in a stage of determining the extraction position for the vibration information and in a stage of extracting the vibration information.

8. A receiver that includes a plurality of receiving antenna elements and receives reflected waves or scattered waves of high frequency signals that are transmitted from a transmitter including a plurality of transmitting antenna elements and are reflected or scattered by a measurement target, the receiver comprising:
a signal conversion unit to convert the reflected waves or the scattered waves of the high frequency signals received by the plurality of the receiving antenna elements into received signals in a frequency band of a radar signal, which is used for generation of the high frequency signal by the transmitter, by using a carrier signal used for generation of the high frequency signal by the transmitter;
a detection unit to detect the received signals using the radar signal and obtain received information that is the reflected waves or the scattered waves of the high frequency signals received by the receiving antenna elements and includes the high frequency signals transmitted from the plurality of the transmitting antenna elements;
a correlation processing unit to perform correlation processing on the received information by using a code used when the radar signal is encoded by the transmitter, and separate the received signals into the individual signals of the transmitting antenna elements transmitted from the transmitter for each of the receiving antenna elements;
a channel reproduction unit to generate channel information indicating a state of a channel between the transmitter and the receiver using the signals separated;
a layer clipping unit to specify a position of the measurement target by extracting, from the channel information, reflection point information of a layer corresponding to a distance in a depth direction of the measurement target as viewed from the plurality of the receiving antenna elements;
a focus correction unit to generate an image of the measurement target by performing focus correction on the measurement target after the position of the measurement target is specified;
a position determination unit to determine an extraction position for vibration information of the measurement target by using the image; and
a vibration information extraction unit to extract the vibration information of the measurement target from the channel information by using the extraction position.

9. The receiver according to claim 8, comprising
a filter to perform filtering on the vibration information and extract, from the vibration information, vibration information about vibration having a desired period.

10. The receiver according to claim 8 or 9, wherein
the position determination unit determines the extraction position optimal for extracting the vibration information of a plurality of the measurement targets from the image for each of the measurement targets, and
the vibration information extraction unit extracts, from the channel information, the vibration information of the plurality of the measurement targets by using the extraction position for each of the measurement targets determined by the position determination unit.

11. A receiver in a sensing system including a plurality of transmitters and a plurality of the receivers, the receiver including a plurality of receiving antenna elements and receiving reflected waves or scattered waves of high frequency signals that are transmitted from the transmitters each including a plurality of transmitting antenna elements and are reflected or scattered by a measurement target, the receiver comprising:
a signal conversion unit to convert the reflected waves or the scattered waves of the high frequency signals received by the plurality of the receiving antenna elements into received signals in a frequency band of a radar signal, which is used for generation of the high frequency signal by the transmitters, by using a carrier signal used for generation of the high frequency signal by the transmitters;
a detection unit to detect the received signals using the radar signal and obtain received information that is the reflected waves or the scattered waves of the high frequency signals received by the receiving antenna elements and includes the high frequency signals transmitted from the plurality of the transmitting antenna elements;
a correlation processing unit to perform correlation processing on the received information by using a code used when the radar signal is encoded by the transmitters, and separate the received signals into the individual signals of the transmitting antenna elements transmitted from the transmitters for each of the receiving antenna elements;
a channel reproduction unit to generate channel information indicating a state of a channel between the transmitters and the receivers using the signals separated;
a layer clipping unit to specify a position of the measurement target by extracting, from the channel information, reflection point information of a layer corresponding to a distance in a depth direction of the measurement target as viewed from the plurality of the receiving antenna elements; and
a focus correction unit to generate an image of the measurement target by performing focus correction on the measurement target after the position of the measurement target is specified, wherein
the channel reproduction unit outputs the channel information to a vibration information extraction device including a position determination unit and a vibration information extraction unit, the position determination unit determining an extraction position for vibration information of the measurement target by using the image, the vibration information extraction unit extracting the vibration information of the measurement target from the channel information by using the extraction position, and
the focus correction unit outputs the image to the vibration information extraction device.

12. A control circuit for controlling a sensing system that includes a transmitter including a plurality of transmitting antenna elements and a receiver including a plurality of receiving antenna elements, the control circuit causing the sensing system to execute:
controlling timings of generation of a radar signal, generation of a code for the receiver to separate high frequency signals transmitted from the plurality of the transmitting antenna elements into the high frequency signals transmitted from individual ones of the transmitting antenna elements, and generation of a carrier signal such that a frequency band available is divided into a plurality of subbands and the subbands used for the high frequency signals transmitted from the plurality of the transmitting antenna elements are periodically switched so that an entire range of the frequency band is used; multiplying the radar signal and the code for each of the plurality of the transmitting antenna elements; generating the high frequency signal having a bandwidth of the subband using the radar signal multiplied by the code and the carrier signal; transmitting the high frequency signal from each of the plurality of the transmitting antenna elements;
receiving the high frequency signals transmitted from the transmitter and reflected or scattered by a measurement target with the plurality of the receiving antenna elements included; generating channel information indicating a state of a channel between the transmitter and the receiver using the carrier signal, the radar signal, and the code; specifying a position of the measurement target using the channel information; generating an image of the measurement target by performing focus correction on the measurement target; determining an extraction position for vibration information of the measurement target by using the image; and extracting the vibration information of the measurement target from the channel information by using the extraction position.

13. A control circuit for controlling a receiver that includes a plurality of receiving antenna elements and receives reflected waves or scattered waves of high frequency signals that are transmitted from a transmitter including a plurality of transmitting antenna elements and are reflected or scattered by a measurement target, the control circuit causing the receiver to execute:
converting the reflected waves or the scattered waves of the high frequency signals received by the plurality of the receiving antenna elements into received signals in a frequency band of a radar signal, which is used for generation of the high frequency signal by the transmitter, by using a carrier signal used for generation of the high frequency signal by the transmitter;
detecting the received signals using the radar signal and obtaining received information that is the reflected waves or the scattered waves of the high frequency signals received by the receiving antenna elements and includes the high frequency signals transmitted from the plurality of the transmitting antenna elements;
correlation processing on the received information by using a code used when the radar signal is encoded by the transmitter, and separating the received signals into the individual signals of the transmitting antenna elements transmitted from the transmitter for each of the receiving antenna elements;
generating channel information indicating a state of a channel between the transmitter and the receiver using the signals separated;
specifying a position of the measurement target by extracting, from the channel information, reflection point information of a layer corresponding to a distance in a depth direction of the measurement target as viewed from the plurality of the receiving antenna elements;
generating an image of the measurement target by performing focus correction on the measurement target after the position of the measurement target is specified;
determining an extraction position for vibration information of the measurement target by using the image; and
extracting the vibration information of the measurement target from the channel information by using the extraction position.

14. A storage medium storing a program for controlling a sensing system that includes a transmitter including a plurality of transmitting antenna elements and a receiver including a plurality of receiving antenna elements, wherein
the program causes the sensing system to execute:
controlling timings of generation of a radar signal, generation of a code for the receiver to separate high frequency signals transmitted from the plurality of the transmitting antenna elements into the high frequency signals transmitted from individual ones of the transmitting antenna elements, and generation of a carrier signal such that a frequency band available is divided into a plurality of subbands and the subbands used for the high frequency signals transmitted from the plurality of the transmitting antenna elements are periodically switched so that an entire range of the frequency band is used;
multiplying the radar signal and the code for each of the plurality of the transmitting antenna elements; generating the high frequency signal having a bandwidth of the subband using the radar signal multiplied by the code and the carrier signal; transmitting the high frequency signal from each of the plurality of the transmitting antenna elements;
receiving the high frequency signals transmitted from the transmitter and reflected or scattered by a measurement target with the plurality of the receiving antenna elements included; generating channel information indicating a state of a channel between the transmitter and the receiver using the carrier signal, the radar signal, and the code; specifying a position of the measurement target using the channel information; generating an image of the measurement target by performing focus correction on the measurement target; determining an extraction position for vibration information of the measurement target by using the image; and extracting the vibration information of the measurement target from the channel information by using the extraction position.

15. A storage medium storing a program for controlling a receiver that includes a plurality of receiving antenna elements and receives reflected waves or scattered waves of high frequency signals that are transmitted from a transmitter including a plurality of transmitting antenna elements and are reflected or scattered by a measurement target, wherein
the program causes the receiver to execute:
converting the reflected waves or the scattered waves of the high frequency signals received by the plurality of the receiving antenna elements into received signals in a frequency band of a radar signal, which is used for generation of the high frequency signal by the transmitter, by using a carrier signal used for generation of the high frequency signal by the transmitter;
detecting the received signals using the radar signal and obtaining received information that is the reflected waves or the scattered waves of the high frequency signals received by the receiving antenna elements and includes the high frequency signals transmitted from the plurality of the transmitting antenna elements;
correlation processing on the received information by using a code used when the radar signal is encoded by the transmitter, and separating the received signals into the individual signals of the transmitting antenna elements transmitted from the transmitter for each of the receiving antenna elements;
generating channel information indicating a state of a channel between the transmitter and the receiver using the signals separated;
specifying a position of the measurement target by extracting, from the channel information, reflection point information of a layer corresponding to a distance in a depth direction of the measurement target as viewed from the plurality of the receiving antenna elements;
generating an image of the measurement target by performing focus correction on the measurement target after the position of the measurement target is specified;
determining an extraction position for vibration information of the measurement target by using the image; and
extracting the vibration information of the measurement target from the channel information by using the extraction position.

16. A sensing method comprising:
a transmission step in which a transmitter including a plurality of transmitting antenna elements performs:
controlling timings of generation of a radar signal, generation of a code for a receiver to separate high frequency signals transmitted from the plurality of the transmitting antenna elements into the high frequency signals transmitted from individual ones of the transmitting antenna elements, and generation of a carrier signal such that a frequency band available is divided into a plurality of subbands and the subbands used for the high frequency signals transmitted from the plurality of the transmitting antenna elements are periodically switched so that an entire range of the frequency band is used;
multiplying the radar signal and the code for each of the plurality of the transmitting antenna elements; generating the high frequency signal having a bandwidth of the subband using the radar signal multiplied by the code and the carrier signal; and transmitting the high frequency signal from each of the plurality of the transmitting antenna elements; and
a reception step in which the receiver including a plurality of receiving antenna elements performs: receiving the high frequency signals transmitted from the transmitter and reflected or scattered by a measurement target; generating channel information indicating a state of a channel between the transmitter and the receiver using the carrier signal, the radar signal, and the code; specifying a position of the measurement target using the channel information; generating an image of the measurement target by performing focus correction on the measurement target; determining an extraction position for vibration information of the measurement target by using the image;
and extracting the vibration information of the measurement target from the channel information by using the extraction position.

17. A receiving method by a receiver that includes a plurality of receiving antenna elements and receives reflected waves or scattered waves of high frequency signals that are transmitted from a transmitter including a plurality of transmitting antenna elements and are reflected or scattered by a measurement target, the receiving method comprising:
a signal conversion step in which a signal conversion unit converts the reflected waves or the scattered waves of the high frequency signals received by the plurality of the receiving antenna elements into received signals in a frequency band of a radar signal, which is used for generation of the high frequency signal by the transmitter, by using a carrier signal used for generation of the high frequency signal by the transmitter;
a detection step in which a detection unit detects the received signals using the radar signal and obtains received information that is the reflected waves or the scattered waves of the high frequency signals received by the receiving antenna elements and includes the high frequency signals transmitted from the plurality of the transmitting antenna elements;
a correlation processing step in which a correlation processing unit performs correlation processing on the received information by using a code used when the radar signal is encoded by the transmitter, and separates the received signals into the individual signals of the transmitting antenna elements transmitted from the transmitter for each of the receiving antenna elements;
a channel reproduction step in which a channel reproduction unit generates channel information indicating a state of a channel between the transmitter and the receiver using the signals separated;
a layer clipping step in which a layer clipping unit specifies a position of the measurement target by extracting, from the channel information, reflection point information of a layer corresponding to a distance in a depth direction of the measurement target as viewed from the plurality of the receiving antenna elements;
a focus correction step in which a focus correction unit generates an image of the measurement target by performing focus correction on the measurement target after the position of the measurement target is specified;
a position determination step in which a position determination unit determines an extraction position for vibration information of the measurement target by using the image; and
a vibration information extraction step in which a vibration information extraction unit extracts the vibration information of the measurement target from the channel information by using the extraction position.
